(19) Europäisches Patentamt
European Patent Office
Office européen des brevets

(11) Veröffentlichungsnummer: **0 081 743**
**B1**

(12) **EUROPÄISCHE PATENTSCHRIFT**

(45) Veröffentlichungstag der Patentschrift: **14.05.86**

(51) Int. Cl.⁴: **C 07 C 127/19**, A 01 N 47/30

(21) Anmeldenummer: **82111090.5**

(22) Anmeldetag: **01.12.82**

# (54) Naphthoxyphenylharnstoffe, Verfahren zu ihrer Herstellung und ihre Verwendung zur Bekämpfung unerwünschten Pflanzenwuchses.

(30) Priorität: **10.12.81 DE 3148914**

(43) Veröffentlichungstag der Anmeldung: **22.06.83 Patentblatt 83/25**

(45) Bekanntmachung des Hinweises auf die Patenterteilung: **14.05.86 Patentblatt 86/20**

(84) Benannte Vertragsstaaten: **BE DE FR GB IT NL**

(56) Entgegenhaltungen: **DE - A - 2 853 791**

***Die Akte enthält technische Angaben, die nach dem Eingang der Anmeldung eingereicht wurden und die nicht in dieser Patentschrift enthalten sind.***

(73) Patentinhaber: **BASF Aktiengesellschaft, Carl-Bosch-Strasse 38, D-6700 Ludwigshafen (DE)**

(72) Erfinder: **Becker, Rainer, Dr., Im Haseneck 22, D-6702 Bad Duerkheim (DE)**
Erfinder: **Goetz, Norbert, Dr., Schoefferstrasse 25, D-6520 Worms 1 (DE)**
Erfinder: **Wuerzer, Bruno, Dr. Dipl.-Landwirt, Ruedigerstrasse 13, D-6701 Otterstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 081 743 B1

**Beschreibung**

Die Erfindung betrifft Naphthoxyphenylharnstoffe, Verfahren zu ihrer Herstellung, Herbizide, die diese Verbindungen als Wirkstoffe enthalten, sowie ein Verfahren zur Bekämpfung unerwünschten Pflanzenwuchses mit diesen Wirkstoffen.

Aus der DE-OS 2 853 791 ist bekannt, dass N′-[4-(2-Naphthoxy)-phenyl]-N,N-dimethyl-harnstoffe und -N-methoxy-N-methyl-harnstoffe herbizid wirksam sind, dass diese Harnstoffe aber dem N′-[4-(1-Naphthoxy)-phenyl]-N′,N′-dimethylharnstoff hinsichtlich der herbiziden Wirksamkeit und der Selektivität überlegen sind.

Es wurde gefunden, dass Naphthoxyphenylharnstoffe der Formel

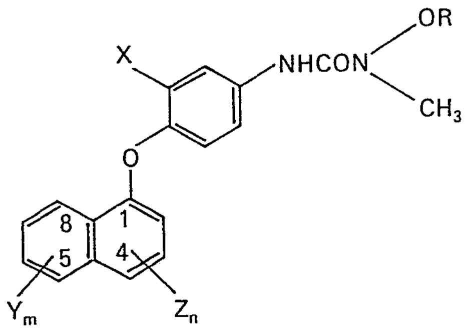

(I),

in der
R Alkyl mit 1 bis 4 Kohlenstoffatomen,
X Wasserstoff, Halogen oder Trifluormethyl und
Y und Z jeweils Wasserstoff, Halogen, Alkyl oder Alkoxy mit jeweils 1 bis 4 Kohlenstoffatomen, und
m und n 1 oder 2 bedeuten,
überraschenderweise gute herbizide und für bestimmte Kulturpflanzen besonders selektive Eigenschaften haben.

In Formel I haben die Substituenten die folgenden Bedeutungen:
R bedeutet Alkyl mit 1 bis 4 Kohlenstoffatomen, wie Methyl, Ethyl, vorzugsweise Methyl,
X bedeutet ausser Wasserstoff oder Trifluormethyl Halogen, wie Chlor oder Brom,
Y und Z bedeuten ausser Wasserstoff Halogen, wie Chlor oder Brom, Alkyl oder Alkoxy mit jeweils 1 bis 4 Kohlenstoffatomen, wie Methyl, Methoxy, Ethyl, Ethoxy, Isopropyl.

Bevorzugte Naphthoxyphenylharnstoffe der Formel I sind solche, bei denen R Methyl und X Chlor oder Trifluormethyl bedeuten, insbesondere N′-[4-(1-Naphthoxy)-3-chlor-phenyl]-N-methoxy-N-methyl-harnstoff.

Man erhält die Naphthoxyphenylharnstoffe der Formel I durch Umsetzung von 1-Naphtholderivaten der Formel

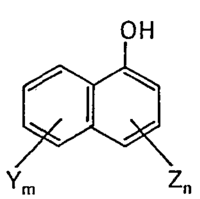

(II),

in der Y, Z, m und n die obengenannten Bedeutungen haben, mit 4-Halogen-nitrobenzolderivaten der Formel

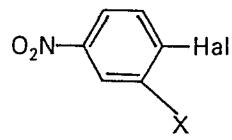

(III),

in der X die obengenannten Bedeutungen hat und Hal für Halogen steht, anschliessende Reduktion des Reaktionsproduktes und Umsetzung des so erhaltenen Amins mit N-Alkoxy-N-methylcarbaminsäurechlorid oder mit Phosgen und N-Alkoxy-N-methylamin zum Harnstoff.

Die Umsetzung der 1-Naphtholderivate der Formel II mit den 4-Halogen-nitro-benzolderivaten der Formel III wird in einem unter den Reaktionsbedingungen inerten Lösungsmittel in Gegenwart einer Base durchgeführt.

Geeignet sind polare Lösungsmittel, beispielsweise Nitrile, wie Acetonitril, Amide, wie Dimethylformamid, oder Dimethylsulfoxid oder Wasser. Auch Gemische dieser Lösungsmittel können verwendet werden.

Wird die Reaktion in wässrigem Medium durchgeführt, wird zweckmässigerweise ein Phasentransferkatalysator, wie Tetrabutylammoniumjodid, zugesetzt. Die Menge an Phasentransferkatalysator beträgt 0,01 bis 1 Mol bezogen auf 1 Mol Verbindung der Formel II.

Die Umsetzung wird zweckmässigerweise in Gegenwart einer Base durchgeführt, wobei die Menge an Base, bezogen auf die Verbindung der Formel III, 1,0 bis 2,0 Mol beträgt. Geeignete Basen sind Alkalicarbonate, Alkalihydrogencarbonate, Alkalihydroxide oder tertiäre Amine, wie Natriumcarbonat, Natriumhydrogencarbonat, Kaliumcarbonat, Natriumhydroxid, Kaliumhydroxid, Triethylamin.

Die Reaktionskomponenten werden in äquimolarem Verhältnis umgesetzt. Ein Überschuss der einen oder anderen Komponente stört nicht. Die Reaktionstemperatur liegt zwischen 50 und 150, vorzugsweise zwischen 80 und 135°C.

Der so erhaltene 1-Naphthyl-(4-nitrophenyl)-ether wird anschliessend in an sich üblicher Weise, z.B. durch katalytische Hydrierung mit Raney-Nickel oder mit Palladium auf Kohle oder mit $SnCl_2$ oder Eisenpulver zum Amin reduziert.

Das Amin lässt sich dann in üblicher Weise in den Harnstoff überführen, beispielsweise durch Umsetzung mit N-Alkoxy-N-methyl-carbaminsäurechlorid. Das Amin kann auch phosgeniert und das entstehende Isocyanat mit N-Alkoxy-N-methylamin umgesetzt werden.

Beispiel

576 Gewichtsteile 1-Naphthol werden in 800 Volumenteilen Dimethylformamid gelöst und mit 768 Gewichtsteilen 3,4-Dichlornitrobenzol und 552 Gewichtsteilen wasserfreiem Kaliumcarbonat versetzt. Das Gemisch wird innerhalb von zwei Stunden auf Rückflusstemperatur gebracht

und 20 Stunden bei dieser Temperatur gerührt. Anschliessend wird gekühlt und zu Eiswasser eingerührt. Man erhält 1180 Gewichtsteile 3-Chlor-4-(1-naphthoxy)-nitrobenzol vom Schmp. 69 bis 74°C.

$C_{16}H_{10}NClO_3$ (M = 300)

Ber.: C 64,12 H 3,36 N 4,67 Cl 11,83
Gef.: C 64,3 H 3,3 N 4,7 Cl 11,7

230 Gewichtsteile 3-Chlor-4-(1-naphthoxy)-nitrobenzol werden in 1500 Volumenteilen Tetrahydrofuran aufgenommen und mit 10 g Palladium/Kohlen (10%ig) versetzt und dann bei 40°C und 100 bar Wasserstoff 12 Stunden hydriert. Anschliessend wird abfiltriert, und das Filtrat wird eingeengt. Es verbleiben 206 Gewichtsteile 3-Chlor-4-(1-naphthoxy)-anilin als hochviskoses Öl, das direkt weiter eingesetzt oder mit wässriger Salzsäure in das feste Hydrochlorid vom Schmp. 210 bis 213°C überführt werden kann.

$C_{16}H_{13}NCl_2O$ (M = 306)

Ber.: C 62,76 H 4,28 N 4,57 Cl 23,16
Gef.: C 62,7 H 4,4 N 4,5 Cl 22,0

15,3 Gewichtsteile 3-Chlor-4-(1-naphthoxy)-anilinhydrochlorid werden in 150 Volumenteilen Tetrahydrofuran aufgenommen und mit 11,1 Gewichtsteilen Triethylamin versetzt. Bei 20°C werden anschliessend 6,8 Gewichtsteile N-Methoxy-N-methylcarbaminsäurechlorid zugetropft. Man lässt 20 Stunden nachrühren, gibt in Eiswasser und saugt den erhaltenen N-[3-Chlor-4-(alpha-naphthoxy-)phenyl-]-N'-methyl-N'-methoxyharnstoff ab. Ausbeute: 16,92 Gewichtsteile; Schmp. 124 bis 127°C.

$C_{19}H_{17}N_2ClO_3$ (M = 357)

Die folgenden Naphthoxyharnstoffe der Formel I können auf analogem Wege hergestellt werden:

| Nr. | X | Y | Z | R | Fp (°C) |
|---|---|---|---|---|---|
| 1 | Cl | H | H | $OCH_3$ | 124–127 |
| 2 | $CF_3$ | H | H | $OCH_3$ | 122–125 |
| 3 | H | H | H | $OCH_3$ | 86– 89 |
| 4 | Cl | H | 4-Cl | $OCH_3$ | 146–148 |
| 5 | H | 5-$OCH_3$ | H | $OCH_3$ | |
| 6 | $CF_3$ | H | 2,4-$(CH_3)_2$ | $OCH_3$ | |
| 7 | H | H | 2-$CH_3$ | $OCH_3$ | |
| 8 | H | H | 4-Cl | $OCH_3$ | 123–126 |
| 9 | Cl | H | 2-$CH_3$ | $OCH_3$ | |
| 10 | $CF_3$ | H | 2-$CH_3$ | $OCH_3$ | |
| 11 | Cl | 5-$OCH_3$ | H | $OCH_3$ | 145–148 |
| 12 | $CF_3$ | H | 4-Cl | $OCH_3$ | 139–141 |
| 13 | Cl | H | 2,4-$(CH_3)_2$ | $OCH_3$ | 126–129 |
| 14 | H | H | 2,4-$(CH_3)_2$ | $OCH_3$ | |
| 15 | Cl | H | 4-$OCH_3$ | $OCH_3$ | |
| 16 | H | H | 4-$OCH_3$ | $OCH_3$ | |
| 17 | $CF_3$ | 5,8-$Cl_2$ | H | $OCH_3$ | |
| 18 | Cl | 5,8-$Cl_2$ | H | $OCH_3$ | |
| 19 | $CF_3$ | H | 4-$OCH_3$ | $OCH_3$ | |
| 20 | Cl | H | H | $OC_2H_5$ | |
| 21 | H | 5,8-$Cl_2$ | H | $OCH_3$ | |
| 22 | $CF_3$ | 5-$OCH_3$ | H | $OCH_3$ | |
| 23 | H | 5-$OC_2H_5$ | H | $OCH_3$ | |
| 24 | $CF_3$ | H | 2-i-$C_3H_7$ | $OCH_3$ | |
| 25 | Cl | H | 2-i-$C_3H_7$ | $OCH_3$ | |
| 26 | Cl | 5-$OC_2H_5$ | H | $OCH_3$ | 113–115 |
| 27 | $CF_3$ | 5-$OC_2H_5$ | H | $OCH_3$ | |
| 28 | H | H | 2-i-$C_3H_7$ | $OCH_3$ | |
| 29 | H | H | 2-$CH_3$ | $OCH_3$ | |
| 30 | $CF_3$ | H | 2-$CH_3$ | $OCH_3$ | |
| 31 | Cl | H | 2-$CH_3$ | $OCH_3$ | |
| 32 | $CF_3$ | H | H | $OC_2H_5$ | |

Die Naphthoxyphenylharnstoffe der Formel I bzw. sie enthaltende herbizide Mittel können beispielsweise in Form von direkt versprühbaren Lösungen, Pulvern, Suspensionen, auch hochprozentigen wässrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Giessen angewendet werden. Die Anwendungsformen richten sich ganz nach den Verwendungszwecken; sie sollten in jedem Fall möglichst die feinste Verteilung der

erfindungsgemässen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wässrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löss, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nussschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind:

I. Man vermischt 90 Gewichtsteile der Verbindung Nr. 1 mit 10 Gewichtsteilen N-Methyl-alpha-pyrrolidon und erhält eine Lösung, die zur Anwendung in Form kleinster Tropfen geeignet ist.

II. 20 Gewichtsteile der Verbindung Nr. 1 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-mono-ethanolamid, 5 Gewichtsteilen Calciumsalz der Dodecyl-benzolsulfonsäure und 5 Gewichtsteilen des Anlagerungsproduktes von 40 Mol Ethylenoxid an 1 Mol Ricinusöl besteht. Durch Ausgiessen und feines Verteilen der Lösung in 100 000 Gewichtsteilen Wasser erhält man eine wässrige Dispersion, die 0,02 Gewichtsprozent des Wirkstoffs enthält.

III. 20 Gewichtsteile der Verbindung Nr. 4 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingiessen und feines Verteilen der Lösung in 100 000 Gewichtsteilen Wasser erhält man eine wässrige Dispersion, die 0,02 Gewichtsprozent des Wirkstoffs enthält.

IV. 20 Gewichtsteile der Verbindung Nr. 12 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingiessen und feines Verteilen der Lösung in 100 000 Gewichtsteilen Wasser erhält man eine wässrige Dispersion, die 0,02 Gewichtsprozent des Wirkstoffs enthält.

V. 20 Gewichtsteile der Verbindung Nr. 8 werden mit 3 Gewichtsteilen des Natriumsalzes der Diisobutylnaphthalin-alpha-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

VI. 3 Gewichtsteile der Verbindung Nr. 11 werden mit 97 Gewichtsteilen feinteiligem Kaolin vermischt. Man erhält auf diese Weise ein Stäubemittel, das 3 Gewichtsprozent des Wirkstoffs enthält.

VII. 30 Gewichtsteile der Verbindung Nr. 13 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile der Verbindung Nr. 26 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Verbindungen der Formel I können im Vor- und Nachauflaufverfahren, vorzugsweise im Nachauflaufverfahren, angewandt werden. Die Anwendung kann aber auch erfolgen, wenn die Kulturpflanzen schon etabliert sind, wie z.B. bei Pflanzreis, die Unkräuter und Ungräser aber noch nicht aufgelaufen sind oder wenn diese gerade dabei sind aufzulaufen. Sind gewisse, bereits stehende Kulturpflanzen gegenüber den Wirkstoffen empfindlich, so können auch Ausbringungstechniken angewandt werden, bei welchen die herbiziden Mittel mit Hilfe der Spritzgeräte so geleitet werden, dass die Blätter empfindlicher Kulturpflanzen nach Möglichkeit nicht getroffen werden, während sie auf die unbedeckte Bodenfläche gelangen und die darin keimenden und aufwachsenden unerwünschten Pflanzen erreichen (post-directed, lay-by, Unterblattspritzung).

In Anbetracht der guten Verträglichkeit und der Vielseitigkeit der Applikationsmethoden und Formulierungsmöglichkeiten können die erfindungsgemässen Herbizide in einer grossen Zahl von Kulturpflanzen zur Beseitigung von unerwünschten Pflanzen eingesetzt werden. Die Aufwandmengen können dabei je nach Bekämpfungsvorhaben, Entwicklungsstadium der Pflanzen und Witterungsverhältnissen zwischen 0,1 und 10 kg/ha und mehr, vorzugsweise zwischen 0,25 bis 2,0 kg Wirkstoff/ha.

In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuss |
| Asparagus officinalis | Spargel |
| Beta vulgaris spp. altissima | Zuckerrübe |
| Beta vulgaris spp. rapa | Futterrübe |
| Beta vulgaris spp. esculenta | Rote Rübe |
| Brassica napus var. napobrassica | Kohlrübe |
| Brassica napus var. rapa | Weisse Rübe |
| Camellia sinensis | Teestrauch |
| Carthamus tinctorius | Saflor, Färberdistel |
| Carya illinoinensis | Pekannussbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süsskartoffel |
| Juglans regia | Walnussbaum |
| Lens culinaris | Linse |
| Linum usitatissimum | Faserlein |
| Lycopersiocon lycopersicum | Tomate |
| Malus spp. | Apfel |
| Manihot esculenta | Maniok |
| Medicago sativa | Luzerne |
| Mentha piperita | Pfefferminze |

| Botanischer Name | Deutscher Name |
|---|---|
| Musa spp. | Obst- u. Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohne |
| Pennisetum glaucum | Perl- oder Rohrkolbenhirse |
| Petroselinum crispum spp. tuberosum | Wurzelpetersilie |
| Picea abies | Rotfichte |
| Abies alba | Weisstanne |
| Pinus spp. | Kiefer |
| Pisum sativum | Gartenerbse |
| Prunus avium | Süsskirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (S. vulgare) | (Mohrenhirse) |
| Sorghum dochna | Zuckerhirse |
| Theobroma cacao | Kakaobaum |
| Trifolium pratense | Rotklee |
| Triticum aestivum | Weizen |
| Vaccinium carymbosum | Kulturheidelbeere |
| Vaccinium vitis-idaea | Preisselbeere |
| Vicia faba | Pferdebohne |
| Vigna sinensis (V. unguiculata) | Kuhbohne |
| Vitis vinifera | Weinrebe |
| Zea mays | Mais |

Von besonderer Bedeutung ist die Bekämpfung von breitblättrigen Unkräutern in Sojakulturen bei Nachauflaufanwendung der Naphthoxyharnstoffe der Formel I, wobei insbesondere folgende Arten zu bekämpfen sind: Abutilon theophrasti (chinesischer Hanf), Amaranthus spp. (Fuchsschwanzarten), Bidens pilosa, Cassia tora, Chenopodium album (weisser Gänsefuss), Datura stramonium (gemeiner Stechapfel), Euphorbia spp. (Wolfsmilcharten), Ipomoea spp. (Prunkwindearten), Sesbania exaltata (Turibaum), Sida spinosa, Sinapis spp. (Senf), Solanum spp. (Nachtschatten).

Den Einfluss der neuen Naphthoxyphenylharnstoffe auf das Wachstum von erwünschten und unerwünschten Pflanzen wird anhand von Gewächshausversuchen gezeigt:

Als Kulturgefässe dienen Plastikblumentöpfe mit 300 $cm^3$ Inhalt und lehmigem Sand mit etwa 1,5% Humus als Substrat. Die Samen der Testpflanzen werden nach Arten getrennt flach eingesät. Unmittelbar danach erfolgt bei Vorauflaufbehandlung das Aufbringen der Wirkstoffe bzw. der herbiziden Mittel auf die Erdoberfläche. Sie werden hierzu in Wasser als Verteilungsmittel suspendiert oder emulgiert und mittels fein verteilender Düsen gespritzt. Die Aufwandmengen betragen 3,0 kg Wirkstoff/ha. Nach dem Aufbringen der Mittel beregnet man die Gefässe leicht, um Keimung und Wachstum in Gang zu bringen. Danach deckt man die Gefässe mit durchsichtigen Plastikhauben ab, bis die Pflanzen angewachsen sind. Diese Abdeckung bewirkt ein gleichmässiges Keimen der Testpflanzen, sofern diese nicht durch die Wirkstoffe beeinträchtigt werden.

Zur Nachauflaufbehandlung zieht man die Testpflanzen je nach Wuchsform erst bis zu einer Wuchshöhe von 3 bis 15 cm an und behandelt sie danach. Die für die Nachauflaufanwendung eingesetzten Sojapflanzen werden in einem mit Torfmull (peat) angereicherten Substrat angezogen. Zur Nachauflaufbehandlung werden in die Versuchsgefässe gesät und darin aufgewachsene Pflanzen ausgewählt. Die Aufwandmengen für die Nachauflaufbehandlung variieren je nach Wirkstoff. Sie betragen beispielsweise 0,25 und

3,0 kg Wirkstoff/ha. Eine Abdeckung unterbleibt bei der Nachauflaufbehandlung.

Die Versuchsgefässe werden im Gewächshaus aufgestellt, wobei für wärmeliebende Arten wärmere Bereiche (20 bis 35°C) und für solche gemässigter Klimate 10 bis 20°C bevorzugt werden. Die Versuchsperiode erstreckt sich über 2 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 0 keine Schädigung oder normaler Aufgang und 100 kein Aufgang bzw. völlige Zerstörung zumindest der oberirdischen Sprossteile.

In die Versuche werden folgende Testpflanzen einbezogen: Amaranthus retroflexus (zurückgekrümmter Fuchsschwanz), Cassia tora, Chenopodium album (weisser Gänsefuss), Glycine max (Soja), Ipomoea supp. (Prunkwindearten), Lolium multiflorum (ital. Rayegras), Sorghum bicolor (Mohrenhirse).

Vergleichsmittel ist N-[4-Naphth-2-oxy-3-chlor-phenyl]-N′-methyl-N′-methoxyharnstoff (DE-OS 2 853 791).

Die Gewächshausversuche zeigen, dass beispielsweise die Verbindungen Nr. 4 und 12 bei Vorauflaufanwendung und die Verbindungen Nr. 8, 11, 13 und 26 bei Nachauflaufanwendung von je 3,0 kg Wirkstoff/ha herbizid wirksam sind. Verbindung Nr. 1 hat beispielsweise bei einer Aufwandmenge von 0,25 kg/ha bei Nachauflaufanwendung eine wesentlich bessere Kulturpflanzenverträglichkeit, speziell für Soja und Kultursorghum, als das Vergleichsmittel, während die herbizide Wirksamkeit vergleichbar ist.

Zur Verbreiterung des Wirkspektrums und zur Erzielung synergistischer Effekte können die Verbindungen der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4 H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht.

Ausserdem kann es von Nutzen sein, die Verbindungen der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Es können auch nicht-phytotoxische Öle und Ölkonzentrate zugesetzt werden.

**Patentansprüche**

1. Naphthoxyphenylharnstoffe der Formel

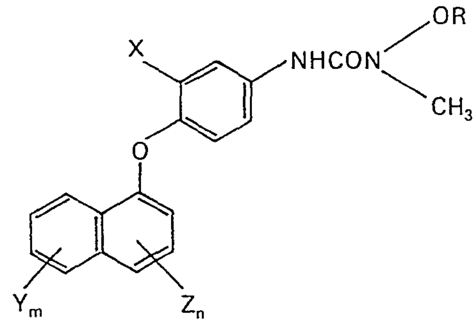

(I),

in der

R Alkyl mit 1 bis 4 Kohlenstoffatomen,

X Wasserstoff, Halogen oder Trifluormethyl und

Y und Z jeweils Wasserstoff, Halogen, Alkyl oder Alkoxy mit jeweils 1 bis 4 Kohlenstoffatomen und

m und n 1 oder 2 bedeuten.

2. N′-[4-(1-Naphthoxy)-3-chlor-phenyl]-N-methoxy-N-methyl-harnstoff.

3. Verfahren zur Herstellung von Naphthoxyphenylharnstoffen der Formel I gemäss Anspruch 1, dadurch gekennzeichnet, dass man ein 1-Naphtholderivat der Formel

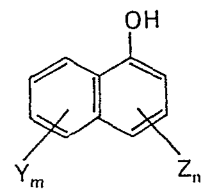

(II),

in der

Y, Z, m und n die im Anspruch 1 genannten Bedeutungen haben, mit einem 4-Halogen-nitrobenzolderivat der Formel

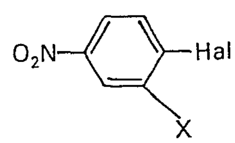

(III),

in der

X die im Anspruch 1 genannte Bedeutung hat und Hal für Halogen steht, in einem unter den Reaktionsbedingungen inerten Lösungsmittel und in Gegenwart eines Säureakzeptors umsetzt, das Reaktionsprodukt in an sich üblicher Weise reduziert und das erhaltene Amin mit N-Alkoxy-N-methylcarbaminsäurechlorid oder mit Phosgen und N-Alkoxy-N-methylamin zum Harnstoff umsetzt.

4. Herbizid, enthaltend einen Naphthoxyphenylharnstoff der Formel I gemäss Anspruch 1.

5. Herbizid, enthaltend inerte Zusatzstoffe und einen Naphthoxyphenylharnstoff der Formel I gemäss Anspruch 1.

6. Herbizid, enthaltend inerte Zusatzstoffe und N′-[4-(1-Naphthoxy)-3-chlor-phenyl]-N-methoxy-N-methylharnstoff.

7. Verfahren zur Bekämpfung unerwünschten Pflanzenwuchses, dadurch gekennzeichnet, dass man die unerwünschten Pflanzen oder die von

unerwünschtem Pflanzenwuchs freizuhaltende Fläche mit einer herbizid wirksamen Menge eines Naphthoxyphenylharnstoffs der Formel I gemäss Anspruch 1 behandelt.

**Claims**

1. A naphthoxyphenylurea of the formula

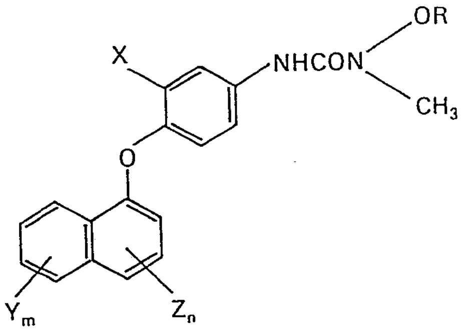

(I),

where
R is alkyl of 1 to 4 carbon atoms,
X is hydrogen, halogen or trifluoromethyl,
Y and Z are each hydrogen, halogen, or alkyl or alkoxy, each of 1 to 4 carbon atoms, and
m and n are each 1 or 2.

2. N′-[4-(1-naphthoxy)-3-chlorophenyl]-N-methoxy-N-methylurea.

3. A process for the preparation of a naphthoxyphenylurea of the formula I as claimed in claim 1, wherein a naphth-1-ol derivative of the formula

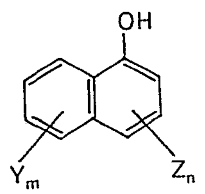

(II),

where
Y, Z, m and n have the meanings given in claim 1, is reacted with a 4-halonitrobenzene derivative of the formula

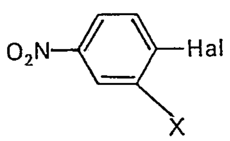

(III),

where
X has the meanings given in claim 1, and Hal is halogen, in a solvent which is inert under the reaction conditions, and in the presence of an acid acceptor; the reaction product is reduced in a conventional manner; and the resulting amine is reacted with N-alkoxy-N-methylcarbamyl chloride, or with phosgene and N-alkoxy-N-methylamine to give the urea.

4. A herbicide containing a naphthoxyphenylurea of the formula I as claimed in claim 1.

5. A herbicide containing inert additives and a naphthoxyphenylurea of the formula I as claimed in claim 1.

6. A herbicide containing inert additives and N′-[4-(1-naphthoxy)-3-chlorophenyl]-N-methoxy-N-methylurea.

7. A process for controlling the growth of unwanted plants, wherein the unwanted plants or the area to be kept free of unwanted plant growth are treated with a herbicidally effective amount of a naphthoxyphenylurea of the formula I as claimed in claim 1.

**Revendications**

1. Naphtoxyphénylurées de formule

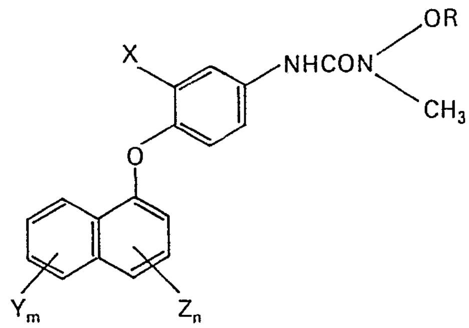

(I),

dans laquelle
R représente alkyle à 1 à 4 atomes de carbone,
X hydrogène, halogène ou trifluorométhyle et
Y et Z chacun, hydrogène, halogène, alkyle ou alcoxy ayant chacun 1 à 4 atomes de carbone et
m et n représentent 1 ou 2.

2. N′-[4-(1-Naphtoxy)-3-chloro-phényl]-N-méthoxy-N-méthylurée.

3. Procédé de préparation de naphtoxyphénylurées de formule I selon la revendication 1, caractérisé par le fait que l'on fait réagir un dérivé de 1-naphtol de formule

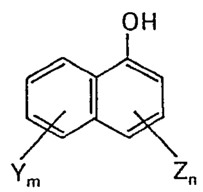

(II),

dans laquelle
Y, Z, m et n ont les significations indiquées dans la revendication 1, avec un dérivé de 4-halogéno-nitrobenzène de formule

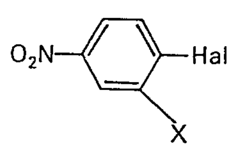

(III),

dans laquelle
X a la signification indiquée dans la revendication 1 et Hal représente halogène, dans un solvant inerte dans les conditions de réaction et en présence d'un accepteur d'acide, on réduit le produit de réaction de manière usuelle et on transforme en urée l'amine obtenue, avec du chlorure d'acide N-alcoxy-N-méthylcarbamique ou avec du phosgène et de la N-alcoxy-N-méthylamine.

4. Herbicide contenant une naphtoxyphénylurée de formule I selon la revendication 1.

5. Herbicide contenant des additifs inertes et naphtoxyphénylurée de formule I selon la revendication 1.

6. Herbicide contenant des additifs inertes et de la N′-[4-(1-naphtoxy)-3-chloro-phényl]-N-méthoxy-N-méthylurée.

7. Procédé de lutte contre la croissance indésirable des plantes, caractérisé par le fait que l'on traite les plantes indésirables ou les surfaces à protéger contre une croissance indésirable des plantes, avec une quantité, efficace du point de vue herbicide, d'une naphtoxyphénylurée de formule I selon la revendication 1.